# EUROPÄISCHE PATENTANMELDUNG

(11) **EP 4 406 521 A1**
(43) Veröffentlichungstag der Anmeldung: **31.07.2024**
(21) Anmeldenummer: 23153736.6
(22) Anmeldetag: 27.01.2023
(51) Int. Cl.: A61F 5/56, A61C 7/08

(54) **ZAHNSCHIENE, SET UMFASSEND EINE MEHRZAHL DERARTIGER ZAHNSCHIENEN UND VERFAHREN ZUR HERSTELLUNG EINER DEARARTIGEN ZAHNSCHIENE**

(71) Anmelder: Adler, Fiona, 82049 Pullach (DE)
(72) Erfinder: Adler, Fiona, 82049 Pullach (DE)
(74) Vertreter: Prüfer & Partner mbB Patentanwälte · Rechtsanwälte

(57) **Zusammenfassung**

Zahnschiene (10), umfassend einen Kunststoffkörper (2) zum intraoralen Tragen auf zumindest einem Teil der Zähne eines Zahnbogens. Der Kunststoffkörper (2) weist eine Sensorhalterung (11), welche ausgebildet ist, einen Sensor (20) reversibel am Kunststoffkörper (2) zu befestigen, auf.

## Beschreibung

Die vorliegende Erfindung betrifft eine Zahnschiene, ein Set umfassend eine Mehrzahl derartiger Zahnschienen und Verfahren zur Herstellung einer derartigen Zahnschiene.

Zur Behandlung von Zahnfehlstellungen werden seit Langem Zahnspangen unterschiedlicher Bauart verwendet.

Gebräuchlich sind beispielsweise festsitzende Zahnspangen, bei denen Klammern (sogenannte "Brackets") auf die Zähne aufgebracht werden. Die Brackets weisen jeweils eine Vorrichtung (das sogenannte "Schloss" oder "Slot") für die Aufnahme von Bögen auf. Ein Aspekt einer derartigen festsitzenden Zahnspange besteht darin, dass die Zahnspange über einen langen Zeitraum von bis zu einigen Jahren ununterbrochen getragen wird und von der Patientin/dem Patienten nicht abgelegt werden kann. Da Zahnspangen als unästhetisch empfunden werden können, empfinden es viele Patientinnen und Patienten als unangenehm oder sogar psychisch belastend, die Zahnspange nicht einmal in Ausnahmesituationen, in denen ihnen das ästhetische Auftreten besonders wichtig ist (gesellschaftliche Anlässe, Fotoaufnahmen etc.), ablegen zu können. Zudem können festsitzende Zahnspangen die Zahnhygiene erschweren, weil sich an den Brackets oder Bögen Verunreinigungen ansammeln können.

Neben den erwähnten festsitzenden Zahnspangen sind herausnehmbare Zahnspangen gebräuchlich, welche als sogenannte "aktive Platten" für den Unterkiefer oder den Oberkiefer ausgebildet sind. Wenn eine aktive Platte getragen wird, übt sie auf die Zähne Kräfte aus, die der Korrektur der Fehlstellung dienen. Eine aktive Platte wird für die Patientin/den Patienten individuell angefertigt. Sie weist üblicherweise einen starren Kunststoffkörper, der beispielsweise aus PMMA besteht, auf. Am Kunststoffkörper befinden sich Halteelemente und aktive Elemente. Als Halteelemente können beispielsweise Dreiecksklammern dienen. Als aktive Elemente, d.h. als die zur Kraftausübung bestimmten Elemente, können beispielsweise Federn, Schrauben oder elastische Elemente dienen. Eine aktive Platte kann üblicherweise verstellt werden, um sie von Zeit zu Zeit an das Behandlungsziel und den Behandlungsfortschritt anzupassen. Da herausnehmbare Zahnspangen nicht fest im Mund der Patientin/des Patienten verankert sind, ist die Compliance ein wesentlicher Faktor für den Erfolg der Behandlung mit einer herausnehmbaren Zahnspange. Unter dem Begriff "Compliance" ist insbesondere die Therapietreue zu verstehen, d.h. die Bereitschaft der Patientin/des Patienten, die Zahnspange so häufig und so lange zu tragen, wie von der Zahnärztin/dem Zahnarzt vorgeschrieben. Aus ästhetischen Gründen, aufgrund eines Störgefühls beim Tragen einer Zahnspange, aus Nachlässigkeit oder aus anderen Gründen tragen viele Patientinnen und Patienten die Zahnspange zu selten oder zu kurz. Dabei ist zu berücksichtigen, dass es sich bei den Patientinnen und Patienten üblicherweise um Kinder oder Jugendliche handelt, die häufig zu einer schlechten Compliance neigen.

Zur Überwachung der Compliance bei der Therapie mit einer herausnehmbaren Zahnspange kann in die Zahnspange ein Sensor, welcher Temperaturwerte erfasst, integriert werden. Der Sensor übermittelt die Messwerte an eine Auswertungsvorrichtung. Die Übermittlung der Messdaten kann dabei kontinuierlich oder nach Zwischenspeicherung im Sensor von Zeit zu Zeit erfolgen. Anhand der Messdaten kann bestimmt werden, wann und wie lange die Patientin/der Patient die Zahnspange im Mund getragen hat. Dabei können neben gemessenen Temperaturwerten an sich auch für das Tragen im Mund typische Temperaturprofile o.ä. als Auswertungskriterium herangezogen werden, um zu verhindern, dass die Patientin/der Patient die Zahnspange zwar nahe am Körper aber nicht im Mund (beispielsweise in der Hosentasche) trägt, um so das Tragen der Zahnspange im Mund vorzutäuschen. Die Übermittlung der Messdaten erfolgt beispielsweise mittels einer Funkverbindung (RFID, Bluetooth o.ä.) zwischen dem Sensor und der Auswertungsvorrichtung oder mittels einer Lesevorrichtung, die in die Nähe des Sensors gebracht wird, um die Messdaten über eine Funkverbindung zu lesen, und die die Messdaten anschließend an die Auswertungsvorrichtung weiterleitet. Bei der Auswertungsvorrichtung kann es sich beispielsweise um einen PC, ein Smartphone, ein Tablet etc. mit einer speziellen Anwendungssoftware oder um eine entsprechende Cloud-Anwendung handeln. Sensoren dieser Art werden beispielsweise von T. C. Schrott und G. Göz im Journal of Orofacial Orthopedics, Band 71, Seiten 339-347 beschrieben.

Im Kontext dieser Patentanmeldung wird unter einem Sensor der beschriebenen Art die gesamte Einheit verstanden, welche neben den zum Erfassen von Messwerten dienenden Komponenten weitere Komponenten umfassen kann. Bei den weiteren Komponenten kann es sich beispielsweise um Batterien sowie um elektronische Komponenten zum Speichern und Übermitteln von Messwerten handeln. Ein Sensor der beschriebenen Art umfasst üblicherweise eine Ummantelung, welche typischerweise flüssigkeitsdicht ist und die Dichtheit gegenüber Speichel und anderen Flüssigkeiten über einen längeren Zeitraum von Monaten oder Jahren gewährleistet. Diese Ummantelung wird ebenfalls als Teil des Sensors aufgefasst.

Im Kontext dieser Patentanmeldung wird unter einem Sensor in erster Linie ein Thermosensor verstanden, d.h. ein Sensor, der Temperaturmesswerte (Temperaturwerte, Temperaturprofile, Temperaturänderungen etc.) erfassen kann. Es ist allerdings möglich, dass der Sensor alternativ oder zusätzlich andere Messwerte erfasst, die alternativ oder zusätzlich zur Kontrolle der korrekten Anwendung einer intraoralen Vorrichtung dienen können. Dabei kann es sich beispielsweise um Abstandswerte, Verformungswerte, Druckwerte, Leitfähigkeitswerte etc. handeln. Mechanische Messwerte (Abstandswerte, Verformungswerte, Druckwerte etc.), die alternativ oder zusätzlich zu Temperaturwerten gemessen werden, können zudem Rückschlüsse auf den korrekten Sitz der intraoralen Vorrichtung und/oder ihre korrekte Anpassung an die Zähne bzw. den Kiefer sowie das Behandlungsziel und den Behandlungsfortschritt erlauben.

Ein relativ neues Produkt zur Korrektur von Zahnfehlstellungen sind Korrekturschienen (sogenannte "Aligner") aus transparentem Kunststoff. Im Gegensatz zu klassischen Zahnspangen besitzen Aligner keine verstellbaren Elemente, sondern es werden nach Erstellung eines Zahnabdrucks oder Kieferabdrucks und Festlegung des Behandlungsziels computergestützt mehrere Zwischenschritte mit einem 3D-Computergrafikverfahren berechnet und eine entsprechende Anzahl transparenter Kunststoffschienen gefertigt. Diese Kunststoffschienen, die als "Aligner", "Aligner-Schienen" etc., bezeichnet werden, werden nacheinander für einen bestimmten Zeitraum von beispielsweise jeweils zwei Wochen getragen. Bei einem typischen Behandlungszeitraum von einem Jahr oder mehr fallen für eine Patientin oder einen Patienten somit Dutzende Zahnschienen an.

Diese Aligner haben die Eigenschaft, weniger sichtbar zu sein als herkömmliche Zahnspangen. Daher wird erwartet, dass die Patientinnen und Patienten diesen Alignern gegenüber weniger ästhetische Vorbehalte haben als gegenüber herkömmlichen Zahnspangen, was zu einer verbesserten Compliance führen sollte.

Die Erfinderinnen haben jedoch festgestellt, dass auch bei der Therapie mit Alignern die Compliance überraschenderweise häufig schlechter ist, als es für einen guten Behandlungserfolg erforderlich wäre. Es handelt sich daher um ein in Fachkreisen verbreitetes Vorurteil, dass Probleme mit der Compliance durch nicht ausreichend häufiges und nicht ausreichend langes Tragen im Fall von Alignern kein schwerwiegendes Problem sei.

S. Kirshenblatt et al., J. Can. Dent. Assoc. 2018;84:i2, Seiten 1-9 beschreiben die Einbettung von Thermosensoren in Retentionsschienen ("Retainer") aus Polymermaterial, wobei die Sensoren jedoch vollständig in Polymermaterial eingebettet sind. In anderen Worten ist der Sensor im Fall dieser Retainerschienen in Kunststoff eingegossen, sodass eine zerstörungsfreie Trennung des Sensors von dem die Zahnschiene bildenden Kunststoffkörper nicht möglich ist.

Ausgehend von der vorstehend beschriebenen Situation besteht eine Aufgabe der vorliegenden Erfindung darin, verbesserte Zahnschienen für die Aligner-Therapie bereitzustellen, welche insbesondere dahingehend verbessert sind, dass sie eine bessere Kontrolle der Compliance gestatten. Eine weitere Aufgabe besteht in der Bereitstellung eines verbesserten Verfahrens zur Herstellung einer Zahnschiene bzw. eines Verfahrens zur Herstellung einer verbesserten Zahnschiene.

Diese Aufgaben werden durch die Zahnschiene gemäß Anspruch 1, das Set gemäß Anspruch 13 und das Verfahren gemäß Anspruch 14 gelöst. Vorteilhafte Ausgestaltungen der vorliegenden Erfindung ergeben sich insbesondere aus den Unteransprüchen. Dabei können die in den Unteransprüchen und in der nachfolgenden Beschreibung des Gegenstands zu einem unabhängigen Anspruch angeführten Merkmale auch zur vorteilhaften Ausgestaltung des Gegenstands zu einem Anspruch derselben oder einer anderen Anspruchskategorie verwendet werden. Beispielsweise kann die Definition eines vorteilhaften Materials, die in einem auf die Zahnschiene bezogenen Unteranspruch angeführt ist, zur Weiterbildung des Verfahrens herangezogen werden, indem dieses Material im entsprechenden Verfahrensschritt gewählt wird.

Ein signifikanter Vorteil der erfindungsgemäßen Zahnschiene besteht darin, dass ein zu diesem Zweck an einer Zahnschiene befestigter Sensor wiederverwendet werden kann, d.h. zusammen mit einer anderen Zahnschiene für denselben oder einen anderen Patienten verwendet werden kann. Dadurch ist eine viel bessere und einfachere Verlaufskontrolle über einen längeren Zeitraum als bisher möglich, und damit wird gleichzeitig die Kontrolle über die Compliance verbessert. Ferner ergeben sich dadurch günstige Kosteneffekte. Ein weiterer Vorteil der vorliegenden Erfindung besteht in der Bereitstellung eines Sets mit einer oder insbesondere mehreren dieser Zahnschienen und einem Sensor, wobei die mehreren Zahnschienen dazu bestimmt sein können, von einer Patientin oder einem Patienten nacheinander verwendet zu werden, und wobei der Sensor gemeinsam mit jeder der mehreren Zahnschienen verwendet werden kann. Weitere Vorteile ergeben sich im Einzelnen aus der nachfolgenden Beschreibung.

Bei der erfindungsgemäßen Zahnschiene handelt es sich um eine Zahnschiene, die einen Kunststoffkörper, welcher dazu bestimmt ist, intraoral, d.h. im Mund, und auf zumindest einem Teil der Zähne eines Zahnbogens getragen zu werden, umfasst. Dazu wird der Kunststoffkörper auf den Zahnbogen bzw. auf die betroffenen Zähne beispielsweise aufgeschoben oder aufgesteckt.

Bevorzugt ist der Kunststoffkörper dazu bestimmt, nicht nur auf einen Teil der Zähne eines Zahnbogens sondern auf allen Zähnen eines Zahnbogens getragen zu werden. Es sind jedoch Anwendungen denkbar, bei denen der Kunststoffkörper so ausgebildet ist, dass er nur auf einem Teil der Zähne eines Zahnbogens zu tragen ist und die übrigen Zähne des Zahnbogens beim intraoralen Tragen der Zahnschiene frei bleiben.

Die vorliegende Erfindung bezieht sich in erster Linie auf Zahnschienen für die Anwendung beim Menschen. Als Zahnbogen wird einer der beiden hufeisenförmig verlaufenden Zahnreihen des Oberkiefers und des Unterkiefers bezeichnet. Für den Zahnbogen sind unter anderem die Bezeichnungen "Alveolarbogen" und "Arcus dentalis" gebräuchlich.

Erfindungsgemäß weist der Kunststoffkörper eine Sensorhalterung auf, wobei die Sensorhalterung ausgebildet ist, einen Sensor reversibel am Kunststoffkörper zu befestigen. Diese Befestigung "am Kunststoffkörper" schließt dabei insbesondere auch eine Art der Befestigung ein, bei der der Sensor zumindest teilweise in einer Vertiefung des Kunststoffkörpers festgehalten wird.

Unter einer reversiblen Befestigung ist eine lösbare Befestigung zu verstehen, d.h. eine Befestigung, die ohne Beschädigung des Sensors, bevorzugt ohne Beschädigung des Sensors und des Kunststoffkörpers, gelöst werden kann.

Dabei ist es möglich, dass eine zum Verschluss der Sensorhalterung dienende Folie, die mit dem Kunststoffkörper verbunden ist, abgelöst oder zertrennt werden muss, bevor der Sensor vom Kunststoffkörper gelöst werden kann.

Dabei ist es ferner möglich, dass der Kunststoffkörper insbesondere im Bereich der Sensorhalterung gebrochen bzw. gerissen wird, wenn der Sensor vom Kunststoffkörper gelöst wird.

In anderen Worten bezieht sich der Begriff der reversiblen Befestigung des Sensors am Kunststoffkörper in erster Linie darauf, dass der mit dem Kunststoffkörper verbundene Sensor wieder vom Kunststoffkörper gelöst werden kann, ohne dass es dabei zu einer Beschädigung des Sensors kommt.

Eine Beschädigung des Kunststoffkörpers kann üblicherweise dann hingenommen werden, wenn es sich bei der Zahnschiene um eine Aligner-Schiene handelt, weil das Lösen des Sensors vom Kunststoffkörper üblicherweise dann erfolgt, wenn die Therapie mit diesem konkreten Kunststoffkörper beendet ist und stattdessen ein anderer Kunststoffkörper zu verwenden ist.

Unter einer Befestigung am Kunststoffkörper wird insbesondere auch eine Befestigung verstanden, bei dem ein Teil des Sensors oder der gesamte Sensor in einer Vertiefung oder einem Hohlraum des Kunststoffkörpers angeordnet ist.

Erfindungsgemäß kann die Zahnschiene einen Sensor, der mittels der Sensorhalterung am Kunststoffkörper befestigt ist, umfassen. Der Sensor ist dann nicht nur am Kunststoffkörper befestigbar sondern tatsächlich am Kunststoffkörper befestigt. In diesem Fall umfasst die Zahnschiene bevorzugt keine weiteren Elemente zusätzlich zum Kunststoffkörper und dem Sensor.

Da die Befestigung reversibel ist, kann ein Sensor, der am Kunststoffkörper befestigt ist, wieder vom Kunststoffkörper gelöst und gemeinsam mit einem anderen Kunststoffkörper, d.h. in einer anderen Zahnschiene, verwendet werden. Durch die Wiederverwendung des Sensors lässt sich der Materialverbrauch in ökonomisch und ökologisch vorteilhafter Weise verringern. Zudem können Arbeitsschritte eingespart werden, die mit der Programmierung eines Sensors verbunden sind, wenn diese nicht für jede neue Zahnschiene erneut durchgeführt werden müssen. Darüber hinaus können die Übertragung und die Auswertung der Messdaten, die von einem Sensor während der Verwendung mit mehreren verschiedenen Kunststoffkörpern aufgezeichnet werden, gemeinsam erfolgen. Das heißt, es muss nicht vor jedem Austausch der Zahnschiene eine separate Datenübertragung und Datenauswertung erfolgen, was den Behandlungsaufwand sowohl für die Patientin/den Patienten als auch für die Zahnärztin/den Zahnarzt verringern kann.

Die beschriebenen Vorteile haben sich als besonders günstig erwiesen, wenn es sich bei den Zahnschienen um Aligner handelt, da die Patientin/der Patient im Fall der Aligner-Therapie hintereinander viele verschiedene Zahnschienen jeweils für einen gewissen Zeitraum von beispielsweise einigen wenigen Wochen trägt.

Wenn der Kunststoffkörper und der mit diesem verbundene Sensor voneinander getrennt werden können, ohne eine dieser beiden Komponenten dabei zu beschädigen, kann auch der Kunststoffkörper wiederverwendet werden. Dies ist beispielsweise dann vorteilhaft, wenn sich herausstellt, dass der mit einem bestimmten Kunststoffkörper verbundene Sensor nicht ordnungsgemäß funktioniert und daher ausgewechselt werden muss. Die Möglichkeit, den Sensor und den Kunststoffkörper so voneinander zu lösen, dass dabei auch der Kunststoffkörper nicht beschädigt wird, hat den weiteren Vorteil, dass zum Herstellen des Kunststoffkörpers mittels Tiefziehen, Gießen oder einem ähnlichen Verfahren ein Kiefermodell mit darauf aufgebrachtem Sensormodell verwendet werden kann, ohne dass beim Trennen von Modell und hergestelltem Kunststoffkörper ein großes Risiko einer Beschädigung des Kunststoffkörpers besteht, weil eine beschädigungsfreie Trennbarkeit des Kunststoffkörpers vom Sensor bedeutet, dass auch der Kunststoffkörper auch vom Sensormodell beschädigungsfrei getrennt werden kann.

Ein weiterer Vorteil der erfindungsgemäßen Zahnschiene besteht darin, dass die erfindungsgemäße Zahnschiene in dem Bereich, in dem der Sensor angeordnet ist, weniger dick ist als es bei einer Zahnschiene der Fall wäre, bei der der Sensor in den Kunststoffkörper eingebettet (z.B. einpolymerisiert oder eingeschmolzen) ist. Daher ruft die erfindungsgemäße Zahnschiene beispielsweise beim Tragen im Mund weniger Störgefühle hervor als es bei einer Zahnschiene, bei der der Sensor in den Kunststoffkörper eingebettet ist, der Fall wäre.

Ein weiterer Vorteil der erfindungsgemäßen Zahnschiene besteht darin, dass die Befestigung des Sensors am Kunststoffkörper nicht nur sicherer ist sondern auch einfacher wieder gelöst werden kann, als es bei einer Zahnschiene der Fall wäre, bei der der Sensor auf den Kunststoffkörper auflaminiert, aufgeschweißt oder aufgeklebt ist.

Bevorzugt handelt es sich beim Kunststoffkörper um einen Überzug, der die Zähne zumindest teilweise bedeckt, wenn die Zahnschiene intraoral getragen wird. Der Kunststoffkörper sitzt dabei nicht zu locker auf den Zähnen sondern ausreichend fest, dass er sich nicht unbeabsichtigt von den Zähnen löst und die Funktionen des Mundes zumindest teilweise möglich sind. Insbesondere soll die Zahnschiene beim Sprechen möglichst wenig stören. In bestimmten Fällen ist es sogar möglich, zu essen, wenn die Zahnschiene intraoral getragen wird.

Bevorzugt ist der Kunststoffkörper zur okklusal bzw. inzisal vollständigen sowie im Übrigen zumindest teilweisen Aufnahme der Zahnkronen aller Zähne eines Zahnbogens ausgebildet. Dies bedeutet, dass die Mahl- und Schneideflächen der Zähne sowie Teile der seitlichen Zahnflächen von der Zahnschiene bedeckt sind, wenn diese intraoral getragen wird.

Weiter bevorzugt nimmt der Kunststoffkörper die Zahnkronen aller Zähne eines Zahnbogens vollständig auf. In anderen Worten umschließt der Kunststoffkörper in diesem Fall die Zahnkronen eines Kiefers vollständig. In diesem Fall kann beispielsweise der Sitz der Zahnschiene besonders sicher sein. Ferner kann in diesem Fall beispielsweise eine zur Korrektur einer Zahnfehlstellung dienende Kraft besonders sicher von der Zahnschiene erzeugt und auf die Zähne übertragen werden.

Bevorzugt handelt es sich bei der erfindungsgemäßen Zahnschiene um eine zur Korrektur von Zahnfehlstellungen ausgebildete Zahnschiene, mehr bevorzugt eine Aligner-Schiene. Abgesehen von einer Aligner-Schiene kann es sich bei einer zur Korrektur von Zahnfehlstellungen ausgebildeten Zahnschiene beispielsweise um eine Positioner-Schiene handeln, d.h. um eine Zahnschiene, die eine Zahn- und Mundstellung, die mithilfe einer Therapie mit einer Zahnspange o.ä. erlangt worden ist, in korrekter Position hält, bis sie sich gefestigt hat.

Da die Patientin/der Patient im Fall der Aligner-Therapie hintereinander viele verschiedene Zahnschienen jeweils für einen gewissen Zeitraum von beispielsweise einigen wenigen Wochen trägt, würden viele Sensoren weggeworfen werden, falls die Zahnschienen jeweils mit einem eigenen Sensor ausgestattet wären.

In anderen bevorzugten Ausführungsbeispielen handelt es sich bei der erfindungsgemäßen Zahnschiene um eine zur Aufhellung von Zähnen ausgebildete Zahnschiene, d.h. um eine sogenannte "Bleaching-Schiene".

In anderen bevorzugten Ausführungsbeispielen handelt es sich bei der erfindungsgemäßen Zahnschiene um eine zur Behandlung von Bruxismus ausgebildete Zahnschiene.

In anderen bevorzugten Ausführungsbeispielen handelt es sich bei der erfindungsgemäßen Zahnschiene um eine zur Behandlung schlafbezogener Atemfehlfunktionen, insbesondere zur Behandlung des Schnarchens und/oder der Schlafapnoe, ausgebildete Zahnschiene.

In anderen bevorzugten Ausführungsbeispielen handelt es sich bei der erfindungsgemäßen Zahnschiene um eine Retentionsschiene.

In anderen bevorzugten Ausführungsbeispielen handelt es sich bei der erfindungsgemäßen Zahnschiene um eine Splint-Zahnschiene, insbesondere zur Anwendung während bzw. nach einer kieferorthopädischen Operation ("OP-Splint").

In anderen bevorzugten Ausführungsbeispielen handelt es sich bei der erfindungsgemäßen Zahnschiene um eine Sport-Performance-Zahnschiene.

In anderen bevorzugten Ausführungsbeispielen handelt es sich bei der erfindungsgemäßen Zahnschiene um eine Mundschutz-Zahnschiene.

Bevorzugt umfasst die Sensorhalterung eine Vertiefung im Kunststoffkörper mit einer auf einer Oberfläche des Kunststoffkörpers angeordneten Öffnung, wobei die Vertiefung zur zumindest teilweisen Aufnahme des Sensors ausgebildet ist. Der Kunststoffkörper weist eine Innenoberfläche auf, die den Zähnen des Zahnbogens zugewandt ist und an diesem sowie gegebenenfalls dem Zahnfleisch anliegt, wenn die Zahnschiene intraoral getragen wird. Der Kunststoffkörper weist ferner eine Außenoberfläche auf, die den Wangen, der Lippe, dem Gaumen bzw. der Zunge und den okklusalen bzw. inzisalen Flächen der Zähne des anderen Zahnbogens zugewandt ist, wenn die Zahnschiene intraoral getragen wird. Die Vertiefung ist dann besonders bevorzugt in einem Abschnitt der Innenoberfläche ausgebildet. Im Fall einer Vertiefung kann die Verbindung des Sensors mit dem Kunststoffkörper beispielsweise und vorteilhaft dadurch erzielt werden, dass zumindest ein Abschnitt des Sensors in die Vertiefung gesteckt wird, was eine einfache, sichere und platzsparende Unterbringung des Sensors darstellt.

Weiter bevorzugt ist die Vertiefung zur vollständigen Aufnahme des Sensors ausgebildet. Dadurch kann der Sensor beispielsweise besonders platzsparend im Kunststoffkörper untergebracht werden. Dadurch kann beispielsweise zudem vermieden werden, dass ein aus dem Kunststoffkörper hervorragender Abschnitt des Sensors den Tragekomfort der Zahnschiene beeinträchtigt.

Insbesondere dann, wenn die die Vertiefung zur vollständigen Aufnahme des Sensors ausgebildet ist, ist die Vertiefung vorzugsweise so ausgebildet, dass ihre Öffnung dem Zahnbogen zugewandt ist, wenn die Zahnschiene intraoral getragen wird. Das bedeutet in anderen Worten, dass die Vertiefung in einem Abschnitt der Innenoberfläche des Kunststoffkörpers ausbildet ist und dann, wenn die Zahnschiene intraoral getragen wird, einen Hohlraum ausbildet, der an einer Seite von Zahnflächen bzw. Zahnfleisch und an den übrigen Seiten vom Material des Kunststoffkörpers umschlossen ist. Dieser Hohlraum bietet Platz für den Sensor und optional für eine Abdeckung in Form eines Verschlusses der Vertiefung (siehe weiter unten). Diese Anordnung der Vertiefung ermöglicht beispielsweise, dass der Kunststoffkörper anhand eines Kiefermodells hergestellt wird, etwa durch Tiefziehen, wobei an das Kiefermodell ein den Abmessungen der Vertiefung entsprechender Formkörper angefügt wird. Der Formkörper entspricht einem Modell des Sensors und umfasst optional einen Abstandshalter (siehe weiter unten).

Im Kontext dieser Patentanmeldung wird unter einem Kiefermodell ein Modell verstanden, welches entsprechend dem Zahnbogen und weiteren anatomischen Details (Form von Abschnitten des Zahnfleischs, des Gaumens etc.) des Oberkiefers oder des Unterkiefers einer Patientin/eines Patienten geformt ist und gegebenenfalls von der tatsächlichen Gestalt dieser Kieferbestandteile abweicht, um eine Korrektur der Zahnstellung bewirken zu können. Ein Kiefermodell kann beispielsweise durch Gießen eines geeigneten Materials (beispielsweise Gips) mithilfe eines von der Patientin/dem Patienten genommenen Alginatabdrucks oder eines vergleichbaren Abdrucks hergestellt werden.

Insbesondere im Fall der Ausbildung der Vertiefung in einem Abschnitt der Innenoberfläche des Kunststoffkörpers ist es besonders bevorzugt, dass die Öffnung der Vertiefung in einem Backenzahnbereich ausgebildet ist, weil der Mund im Bereich der Backenzähne typischerweise mehr Platz für eine Verdickung der Zahnschiene durch den Sensor bietet und die Anordnung in diesem Mundbereich für die Patientin/den Patienten typischerweise weniger störend ist.

Zusätzlich oder alternativ zur Anordnung im Backenzahnbereich ist es bevorzugt, den Sensor bukkal anzuordnen, da eine bukkale Anordnung üblicherweise als weniger störend empfunden wird als eine palatinale bzw. linguale Anordnung. Im Vergleich zu einer palatinalen bzw. lingualen Anordnung ist die Messgenauigkeit eines bukkal angeordneten Sensors, beispielsweise eines bukkal angeordneten Thermosensors, üblicherweise nicht geringer.

Ferner ist es insbesondere im Fall von Aligner-Schien bevorzugt, den Sensor nicht im Bereich derjenigen Zähne anzuordnen, die im Zuge der Aligner-Therapie bewegt werden sollen.

Gemäß bevorzugten Ausführungsbeispielen der vorliegenden Erfindung, die auch die vorstehend beschriebenen bevorzugten Merkmale aufweisen können, ist die Sensorhalterung zur formschlüssigen Verbindung mit dem Sensor ausgebildet. Im Fall einer Vertiefung kann dies vorzugsweise dadurch erreicht werden, dass die Vertiefung folgende Abschnitte aufweist: einen der Form und der Größe des Sensors entsprechenden Aufnahmeabschnitt zur vollständigen Aufnahme des Sensors und einen Öffnungsabschnitt, der zwischen dem Aufnahmeabschnitt und dem Zahnbogen angeordnet ist, wenn die Zahnschiene intraoral getragen wird, wobei bevorzugt der Öffnungsabschnitt so ausgebildet ist, dass der im Aufnahmeabschnitt angeordnete Sensor den Öffnungsabschnitt ohne Verformung des Sensors und/oder des Kunststoffkörpers nicht passieren kann. In diesem Fall kann der Sensor nicht aus der Vertiefung herausrutschen, ohne entsprechend stark verformt zu werden bzw. ohne dass der Kunststoffkörper entsprechend stark verformt wird. Solche Verformungen treten bei der üblichen Verwendung der Zahnschiene, d.h. beim Einsetzen in den Mund, beim Tragen im Mund und beim Herausnehmen aus dem Mund, nicht auf, sodass der Sensor bei der Verwendung sicher im Kunststoffkörper verankert ist. Um den Sensor in den Aufnahmeabschnitt einzubringen oder um den Sensor aus dem Aufnahmeabschnitt zu entnehmen, ist es erforderlich, bewusst eine Verformung vorzunehmen, beispielsweise durch entsprechendes Verbiegen des Kunststoffkörpers, Anwendung einer Kraft zum Hineinpressen des Sensors oder Aushebeln des Sensors mit einem Werkzeug (Spatel, Schraubenzieher, etc.). Die verformten Materialien sind dabei elastisch. Anders ausgedrückt ist der Öffnungsabschnitt gezielt etwas kleiner bemessen, als es den Abmessungen des Sensors entsprechen würde. In anderen Worten ist im Bereich des Öffnungsabschnitts eine Unterschneidung vorgesehen. Diese Unterschneidung in Form einer Verengung gegenüber dem Aufnahmeabschnitt stellt bevorzugt eine Einraststruktur für den Sensor bereit. Die Einraststruktur ist dabei bevorzugt entlang des gesamten Umfangs der Öffnung ausgebildet. Mehr bevorzugt ist die Einraststruktur so ausgebildet, dass sie den Rand eines im Aufnahmeabschnitt angeordneten Sensors dicht einschließt.

Bei der Verformung, die zum Einbringen des Sensors in den Aufnahmeabschnitt oder zum Entnehmen des Sensors aus dem Aufnahmeabschnitt erforderlich ist, handelt es sich bevorzugt zumindest im Wesentlichen um eine Verformung des Kunststoffkörpers. Dadurch wird beim Einbringen oder Entnehmen eine starke Verformung des Sensors, welche zu einer Beschädigung des Sensors führen könnte, vermieden.

In bevorzugten Ausführungsbeispielen der vorliegenden Erfindung, die auch die vorstehend beschriebenen bevorzugten Merkmale aufweisen können, umfasst der Kunststoffkörper einen thermoplastischen Kunststoff. Mehr bevorzugt ist der Kunststoffkörper aus einem thermoplastischen Kunststoff hergestellt. Die Wahl eines thermoplastischen Kunststoffs kann beispielsweise eine einfache Herstellung des Kunststoffkörpers, zum Beispiel durch Tiefziehen an einem Modell, ermöglichen.

Bei dem thermoplastischen Kunststoff handelt es sich weiter bevorzugt um einen thermoplastischen Polyester, ein Polyolefinmaterial, ein thermoplastisches Elastomermaterial, ein thermoplastisches Siliconmaterial oder eine Mischung aus mehreren der vorgenannten Materialien. Ein thermoplastischer Polyester kann dabei ein Homopolymer oder ein Copolymer ("Copolyester") sein. Ein Polyolefinmaterial kann dabei unterschiedliche Polyolefine, ein thermoplastisches Elastomermaterial unterschiedliche thermoplastische Elastomere und ein thermoplastisches Siliconmaterial unterschiedliche Silicone enthalten. Zudem können im thermoplastischen Kunststoff gegebenenfalls geeignete Hilfs- oder Füllstoffe enthalten sein.

Besonders bevorzugt ist es, dass der Kunststoffkörper aus einem Polyester mit oder ohne Hilfs- oder Füllstoffen hergestellt ist, wobei Polyethylenterephthalat-Glycol Copolyester (PET-G) in vielen Fällen besonders geeignet ist, insbesondere PET-G ohne Hilfs- und Füllstoffe.

Mit den vorstehend beschriebenen Kunststoffen können beispielsweise Kunststoffkörper bereitgestellt werden, die die erforderlichen mechanischen Eigenschaften aufweisen und für den intraoralen Einsatz geeignet sind, d.h. nicht gesundheitsschädlich sind und den im Mund herrschenden Bedingungen gut standhalten.

Alternativ oder zusätzlich umfasst der Kunststoffkörper einen Kunststoff mit einem Zug-Elastizitätsmodul nach ISO 527 von 1000 MPa oder weniger, mehr bevorzugt 600 MPa oder weniger, noch mehr bevorzugt 500 MPa oder weniger, wobei der Zug-Elastizitätsmodul nach ISO 527 in den genannten Fällen insbesondere 100 MPa oder mehr beträgt. Dadurch kann beispielsweise ein Kunststoffkörper mit Elastizitätseigenschaften, die für intraorale Anwendungen geeignet sind, bereitgestellt werden, insbesondere ein Kunststoffkörper mit Elastizitätseigenschaften, der eine Korrektur von Zahnfehlstellungen im Rahmen einer Aligner-Therapie erlaubt.

Die vorstehende Angabe der Norm ISO 527 bezieht sich auf ISO 527-1 und ISO 527-2 in den derzeit geltenden Fassungen, d.h. ISO 527-1 :2019-07 und ISO 527-2:2012-02.

Beim Sensor, der gemeinsam mit dem Kunststoffkörper der erfindungsgemäßen Zahnschiene zu verwenden ist, handelt es sich bevorzugt um einen Sensor, dessen funktionelle Komponenten (z.B. Platine und mögliche weitere elektronische Komponenten, Batterie, etc.) durch eine Ummantelung flüssigkeitsdicht umschlossen sind. Bei der Ummantelung kann es sich beispielsweise um ein Polyurethan oder einen anderen geeigneten Kunststoff handeln.

In bevorzugten Ausführungsbeispielen der vorliegenden Erfindung, die auch die vorstehend beschriebenen bevorzugten Merkmale aufweisen können, umfasst die Zahnschiene einen Verschluss zum Verschließen des in der Sensorhalterung angeordneten Sensors. Weiter bevorzugt kann mithilfe des Verschlusses der Aufnahmeabschnitts der als Vertiefung ausgebildeten Sensorhalterung versiegelt werden. Das heißt, dass der Sensor dann gegenüber dem Mund dicht abgeschlossen ist, wenn die Zahnschiene intraoral getragen wird. Auf diese Weise wird beispielsweise vermieden, dass der Sensor in direkten Kontakt mit dem Mund bzw. mit Speichel kommt. Somit können sich in der Vertiefung weder Speichel noch Speisereste ansammeln. Zudem ist die Verwendung eines Sensors möglich, der nicht für den direkten Kontakt mit dem Mund geeignet oder zugelassen ist.

In konkreten bevorzugten Ausführungsbeispielen ist der Verschluss als Pfropfen ausgebildet, der in den Öffnungsabschnitt der Vertiefung einzubringen ist, um die Öffnung abzudichten, nachdem der Sensor in den Aufnahmeabschnitt der Vertiefung eingebracht worden ist. Insbesondere kann dafür ein Kunststoffpfropfen, beispielsweise ein Pfropfen aus dem Material des Kunststoffkörpers oder aus einem weicheren Kunststoffmaterial verwendet werden. Bei Verwendung eines Pfropfens kann der Verschluss beispielsweise besonders einfach entfernt werden, um die Verbindung zwischen dem Kunststoffkörper und dem Sensor zu lösen, da der Pfropfen dazu lediglich aus dem Öffnungsabschnitt der Vertiefung herausgezogen werden muss.

In alternativen Ausführungsbeispielen ist der Verschluss als Folie ausgebildet, die bestimmt ist, den mit dem Kunststoffkörper verbundenen Sensor zu bedecken. Besonders vorteilhaft ist es in diesem Zusammenhang, wenn die Sensorhalterung als Vertiefung ausgebildet ist und die Folie die Öffnung der Vertiefung verschließt, beispielsweise indem sie im Bereich der Öffnung einrastet und/oder rund um die Öffnung mit der Oberfläche des Kunststoffkörpers verklebt oder verschweißt ist. Bei Verwendung einer Folie kann beispielsweise eine besonders sicher abdichtende Versiegelung des Sensors bereitgestellt werden.

Als Folie kann beispielsweise eine Folie aus dem Material des Kunststoffkörpers oder aus einem anderen für die intraorale Anwendung geeigneten Material verwendet werden.

In einem konkreten bevorzugten Ausführungsbeispiel ist die Sensorhalterung als Vertiefung im Kunststoffkörper ausgebildet, wobei die Vertiefung folgende Abschnitte aufweist: einen Aufnahmeabschnitt zur vollständigen Aufnahme des Sensors und einen Öffnungsabschnitt, der zwischen dem Aufnahmeabschnitt und dem Zahnbogen angeordnet ist, wenn die Zahnschiene intraoral getragen wird. Der Öffnungsabschnitt ist gezielt kleiner bemessen, als es den Abmessungen des Sensors entsprechen würde, sodass im Bereich des Öffnungsabschnitts eine Unterschneidung ausgebildet ist. Diese Unterschneidung in Form einer Verengung gegenüber dem Aufnahmeabschnitt stellt bevorzugt eine Einraststruktur für den Sensor bereit. Die Einraststruktur ist dabei bevorzugt entlang des gesamten Umfangs der Öffnung ausgebildet. Die als Verschluss dienende Folie ist zusätzlich zum Sensor so in den Aufnahmeabschnitt eingebracht, dass sie zwischen der Einraststruktur und dem Sensor eingeklemmt ist und den Aufnahmeabschnitt gegenüber dem

Öffnungsabschnitt verschließt. In anderen Worten ist die Folie so im Bereich der Sensorhalterung eingerastet, dass sie den Sensor gegenüber der Umgebung verschließt.

Anstatt einen vorgefertigten Pfropfen oder eine vorgefertigte Folie als Verschluss zu verwenden, kann der Verschluss auch aus einem härtbaren Kunststoff in situ hergestellt werden. Dies erfolgt bevorzugt dadurch, dass die Bereiche des mit der Sensorhalterung verbundenen Sensors, die nicht vom Kunststoffkörper bedeckt sind, mit dem härtbaren Kunststoff bedeckt werden. Der härtbare Kunststoff wird anschließend gehärtet. Zur Initiierung der Härtungsreaktion kann beispielsweise eine Bestrahlung mit Licht oder UV-Strahlung erforderlich sein. Für die In-situ-Herstellung des Verschlusses geeignete Kunststoffmaterialien werden beispielsweise von der Firma SCHEU-DENTAL GmbH in Iserlohn (Deutschland) unter dem Handelsnamen "DURASPLINT" angeboten.

Die kombinierte Verwendung eines Pfropfens und einer Folie ist ebenfalls möglich.

In vorstehender Beschreibung und an anderen Stellen dieser Patentanmeldung werden verschiedene Merkmale des Kunststoffkörpers unter Bezugnahme auf einen Sensor beschrieben. Beispielsweise betrifft dies die Ausgestaltung der Sensorhalterung, bevorzugt in Form einer Vertiefung im Kunststoffkörper mit einem Aufnahmeabschnitt, deren Form und Größe auf den Sensor abgestimmt sind, und einen Öffnungsabschnitt, der eine Einraststruktur bereitstellt. Diese Merkmalsbeschreibung ist so zu verstehen, dass der Kunststoffkörper vorzugsweise gemeinsam mit einem vorbestimmten Sensor zu verwenden ist und die Merkmale des Kunststoffkörpers auf die Form und Größe dieses vorbestimmten Sensors abgestimmt sind.

Alternativ kann der Sensor als Bestandteil der erfindungsgemäßen Zahnschiene verstanden werden. Die Merkmale des Kunststoffkörpers sind in diesem Fall auf die Form und Größe des Sensors, der Bestandteil der Zahnschiene ist, abgestimmt.

Bei dem erfindungsgemäßen Set handelt es sich um ein Set mit einer Mehrzahl an erfindungsgemäßen Zahnschienen (jeweils ohne den Sensor als deren Bestandteil aufzufassen) und einen Sensor. Bevorzugt besteht das Set aus der Mehrzahl an Zahnschienen und genau einem Sensor. In Form dieses Sets können beispielsweise mehrere Zahnschienen, die von der Patientin/dem Patienten nacheinander jeweils für einen bestimmten Zeitraum im Zuge einer Aligner-Therapie zu tragen sind, bereitgestellt werden, wobei der Sensor an jeweils der Zahnschiene, die aktuell zu tragen ist, befestigt wird. Die Merkmale der einzelnen Zahnschienen (d.h. der einzelnen Kunststoffkörper) sind in diesem Fall auf die Form und Größe dieses Sensors abgestimmt.

Bei dem erfindungsgemäßen Verfahren handelt es sich um ein Verfahren zur Herstellung einer Zahnschiene, umfassend die Schritte:
- Bereitstellen eines Kiefermodells,
- Bereitstellen eines Sensormodells,
- Verbinden des Sensormodells mit dem Kiefermodell,
- Bereitstellen eines thermoplastischen Kunststoffmaterials,
- Tiefziehen des thermoplastischen Kunststoffmaterials auf das Kiefermodell mit dem damit verbundenen Sensormodell.

Durch das Verfahren kann insbesondere eine Zahnschiene gemäß vorteilhaften Ausführungsbeispielen der vorliegenden Erfindung hergestellt werden.

Der Schritt des Bereitstellens des Kiefermodells kann auch dessen in der Zahnmedizin und Zahntechnik übliche Herstellung umfassen, beispielsweise in Form eines Gipsgusses auf Grundlage eines Alginat-Abdrucks. Die Form des Kiefermodells kann von den tatsächlichen anatomischen Merkmalen der Patienten/des Patienten abweichen, um eine Korrektur der Zahnstellung bewirken zu können.

Der Schritt des Bereitstellens des Sensormodells kann auch dessen Herstellung umfassen. Dabei kann das Sensormodell beispielsweise auf ähnliche Weise wie das Kiefermodell hergestellt werden, indem ein Abdruck des Sensors hergestellt und das Sensormodell entsprechend diesem Abdruck hergestellt wird.

Zum Tiefzeihen kann ein in der Zahntechnik übliches Tiefziehgerät verwendet werden. Dabei wird das Modell (Kiefermodell mit dem damit verbundenen Sensormodell) auf einer Unterlage, beispielsweise einem Granulat, angeordnet. Auf dem Modell wird eine durch Erwärmen erweichte Folie oder Platte aus dem als Schienenmaterial dienenden thermoplastischen Kunststoff angeordnet. Es wird Unterdruck angelegt, sodass das erweichte Kunststoffmaterial an das Modell gepresst wird. Optional kann das Kiefermodell des anderen Kiefers oder ein anderes geeignetes Werkzeug aufgepresst werden, sodass die Außenoberfläche der Zahnschiene eine zum anderen Zahnbogen passende Form hat. Optional kann vor dem Tiefziehen zwischen dem Schienenmaterial und dem Modell eine Platzhalterfolie angeordnet werden, welche aus einem Material besteht, das sich beim beschriebenen Tiefziehen nicht mit dem Schienenmaterial verbindet. Die Platzhalterfolie kann in einem dem beschriebenen Tiefziehen des Schienenmaterials vorgelagerten Tiefziehschritt ihrerseits durch Anwendung eines Unterdrucks und eventueller Erwärmung auf das Modell gezogen werden. Nach dem Abkühlen des tiefgezogenen Schienenmaterials erfolgen die Entnahme aus dem Tiefziehgerät, das Ablösen des tiefgezogenen Kunststoffs und das Ausschneiden der Zahnschiene, wobei diese Schritte in jeder zweckmäßigen Reihenfolge vorgenommen werden können.

Bevorzugt wird im Schritt des Verbindens des Sensormodells mit dem Kiefermodell das Sensormodell über einen Abstandhalter mit dem Kiefermodell verbunden. Der Abstandshalter kann dabei integral mit dem Sensormodell ausgebildet werden, beispielsweise indem in der vorstehenden Herstellung des Sensormodells anstelle eines Abdrucks des Sensors ein Abdruck vom Sensor, auf dem eine dem Abstandshalter entsprechende Form aufgebracht ist, verwendet wird. Der Abstandshalter kann beispielsweise in Form eines Aufbaus aus einer Modelliermasse (Wachs o.ä.) aufgebracht werden.

Der Abstandshalter führt dazu, dass beim Tiefziehen mit dem Schienenmaterial eine Vertiefung erzeugt wird, die entsprechend dem Sensormodell einen Aufnahmeabschnitt zur Aufnahme des Sensors und entsprechend dem Abstandshalter einen Öffnungsabschnitt, der beim intraoralen Tragen der Zahnschiene zwischen dem Zahnbogen bzw. Zahnfleisch und dem Sensor liegt, aufweist. Bevorzugt springt der Abstandshalter zumindest teilweise gegenüber dem Sensor zurück, sodass sich beim Tiefziehen die weiter oben beschriebene Unterschneidung ausbildet.

Weitere Merkmale, Zweckmäßigkeiten und Vorteile der vorliegenden Erfindung werden nachfolgend anhand von exemplarischen Ausführungsbeispielen unter Bezugnahme auf die angeschlossenen Figuren beschrieben. Die Ausführungsbeispiele beziehen sich auf Zahnschienen, die für die Aligner-Therapie bestimmt sind. Analoge Ausführungsbeispiele ergeben sich für andere Anwendungsfälle, insbesondere die weiter oben beschriebenen Anwendungsfälle, wobei der Kunststoffkörper jeweils hinsichtlich Gestalt und Material entsprechend der Anwendung ausgebildet ist.
- Fig.1: ist eine schematische Darstellung einer herkömmlichen Aligner-Schiene in perspektivischer Schrägansicht.
- Fig. 2: ist eine Darstellung einer Zahnschiene gemäß einem Ausführungsbeispiel der vorliegenden Erfindung in einer perspektivischen Ansicht, die die Innenoberfläche zeigt.
- Fig. 3: ist eine Detaildarstellung eines Abschnitts der in Fig. 2 dargestellten Zahnschiene in einer perspektivischen Ansicht, die die Innenoberfläche zeigt.
- Fig. 4: ist eine Darstellung der in den Fig. 2 und 3 dargestellten Zahnschiene in einer perspektivischen Ansicht, die die Außenoberfläche zeigt.
- Fig. 5: ist eine schematische Schnittdarstellung der in den Fig. 2 bis 4 dargestellten Zahnschiene in der in den Fig. 2 und 3 durch eine gestrichelte Linie symbolisierten Schnittebene.
- Fig. 6: entspricht der Darstellung in Fig. 5, wobei in der Vertiefung der Zahnschiene kein Sensor integriert ist.
- Fig. 7: zeigt eine fotografische Aufnahme der Zahnschiene gemäß dem Ausführungsbeispiel entsprechend der Darstellung in Fig. 2.
- Fig. 8: zeigt eine fotografische Aufnahme der Zahnschiene gemäß dem Ausführungsbeispiel entsprechend der Darstellung in Fig. 3.
- Fig. 9: zeigt eine fotografische Aufnahme der Zahnschiene gemäß dem Ausführungsbeispiel entsprechend der Darstellung in Fig. 4.
- Fig. 10 (a): zeigt den Sensor gemäß einem Ausführungsbeispiel in einer schematischen Schrägansicht.
- Fig. 10 (b): zeigt den in Fig. 10(a) dargestellten Sensor in einer schematischen Seitenansicht.
- Fig. 10 (c): zeigt den in den Fig. 10 (a) und 10 (b) dargestellten Sensor in einer anderen schematischen Seitenansicht.
- Fig. 11: ist eine schematische Schnittdarstellung einer Zahnschiene gemäß einem weiteren Ausführungsbeispiel der vorliegenden Erfindung.
- Fig. 12: ist eine schematische Schnittdarstellung einer Zahnschiene gemäß einem weiteren Ausführungsbeispiel der vorliegenden Erfindung.
- Fig. 13: zeigt eine fotografische Aufnahme eines in einem Verfahren gemäß einem Ausführungsbeispiel der vorliegenden Erfindung zum Tiefziehen verwendeten Modells.

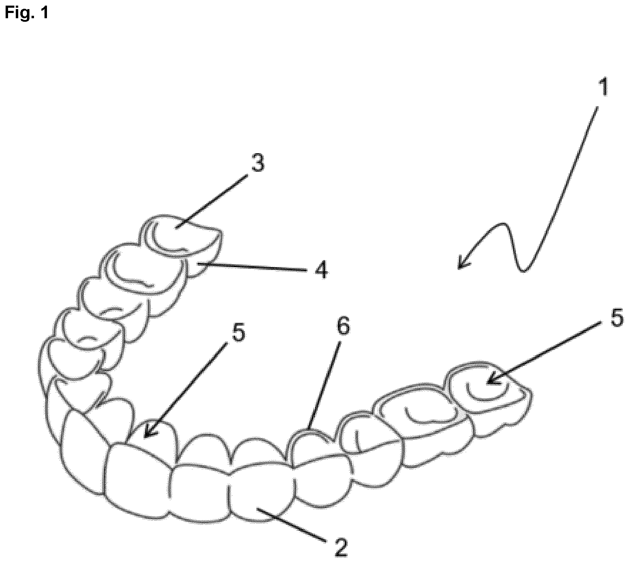

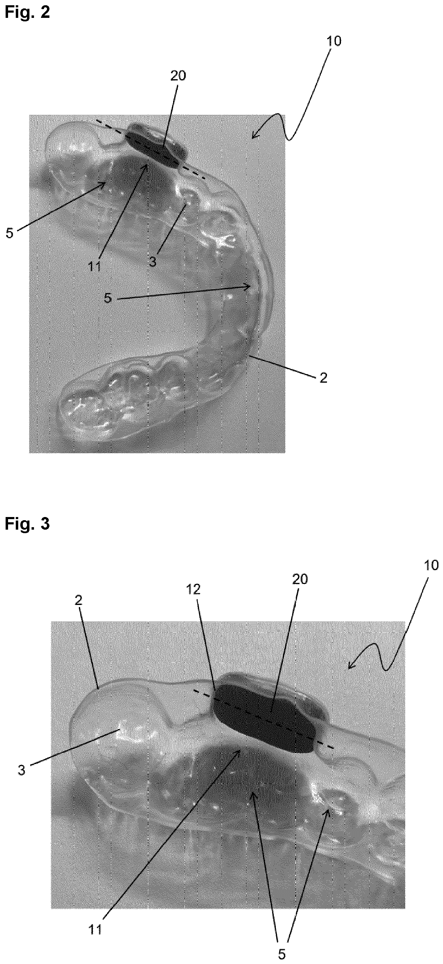

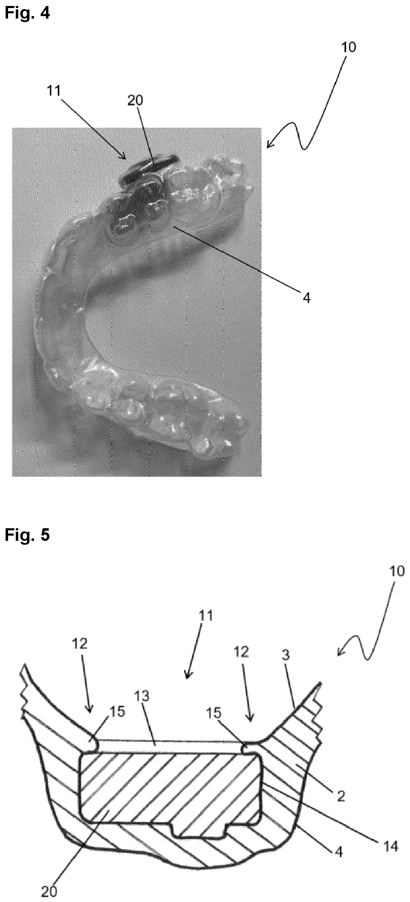

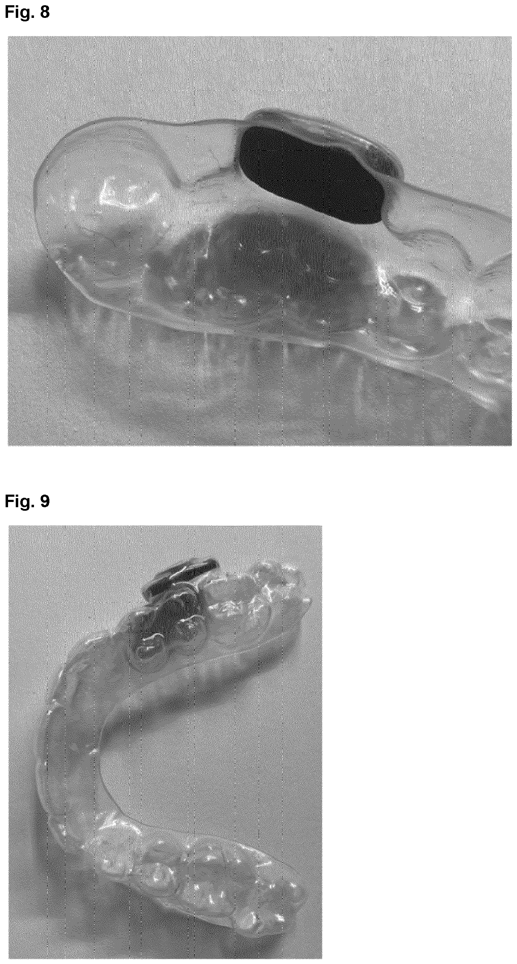

Für Komponenten der in den Figuren 2 bis 9 dargestellten Zahnschiene, die den Komponenten der in Fig. 1 dargestellten Zahnschiene entsprechen, werden mit denselben Bezugszeichen bezeichnet.

In Fig. 1 ist eine im Stand der Technik bekannte konventionelle Zahnschiene 1, welche für die Aligner-Therapie bestimmt ist und daher als "Aligner-Schiene" oder "Aligner" bezeichnet werden kann, schematisch dargestellt.

Diese konventionelle Aligner-Schiene 1 weist einen Kunststoffkörper 2 mit mehreren Ausnehmungen 5 auf. Der Kunststoffkörper 2 ist beispielsweise aus Polyethylenterephthalat-Glycol Copolyester (PET-G) hergestellt. Die Ausnehmungen 5 sind an die Zähne eines der Zahnbögen der Patientin/des Patienten angepasst, sodass die Zähne von den Ausnehmungen 5 aufgenommen werden, wenn die Aligner-Schiene 1 auf den Zahnbogen gesteckt wird. Eine Ausnehmung 5 kann so geformt sein, dass sie genau einen Zahn in sich aufnehmen kann. In anderen Worten können die den Zahnzwischenräumen entsprechenden Trennwände zwischen den Hohlräumen für die einzelnen Zähne teilweise fehlen. Eine Ausnehmung 5 kann auch so geformt sein, dass sie mehrere Zähne in sich aufnehmen kann. Die Ausnehmungen 5 sind so geformt, dass die Aligner-Schiene 1 die Zähne okklusal bzw. inzisal vollständig und die Zahnkronen im Übrigen zumindest teilweise bedeckt. Bevorzugt nehmen die Ausnehmungen 5 der Aligner-Schiene 1 die Zahnkronen aller Zähne eines Zahnbogens vollständig in sich auf, sodass der Rand 6 der Ausnehmungen im Bereich des Übergangs zwischen Zahnkrone und Zahnfleisch oder im Bereich des Zahnfleischs angeordnet ist, wenn die Aligner-Schiene 1 von der Patientin/vom Patienten korrekt im Mund getragen wird. Es ist daher möglich, dass die Aligner-Schiene auch Abschnitte des Zahnfleischs bedeckt. Die Gesamtheit der Oberflächen der Ausnehmungen 5 bildet die Innenoberfläche 3 der Aligner-Schiene, während die übrigen Abschnitte der Oberfläche der Aligner-Schiene 1 dessen Außenoberfläche 4 bildet.

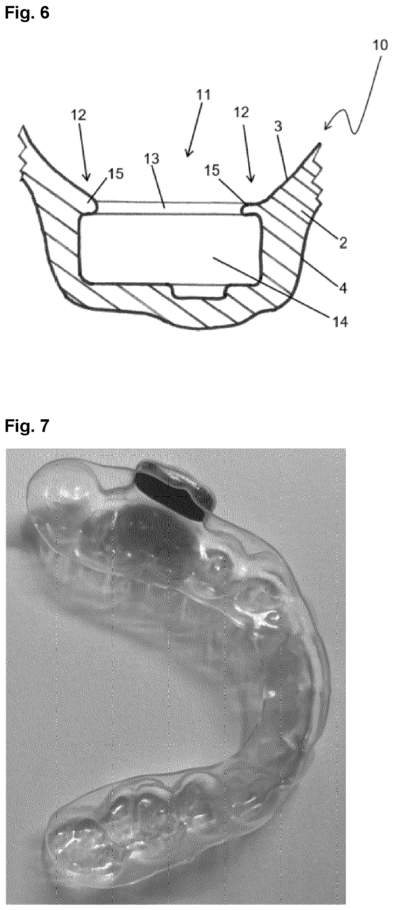

In den Figuren 2 bis 9 ist eine Zahnschiene 10 gemäß einem Ausführungsbeispiel der vorliegenden Erfindung in unterschiedlichen Ansichten und Darstellungen gezeigt. In den Figuren 7 bis 9 ist die Zahnschiene 10 ohne Bezugszeichen fotografisch dargestellt.

Als Zahnschiene 10 gemäß dem Ausführungsbeispiel ist eine Zahnschiene mit integriertem Sensor 20 dargestellt. Als Zahnschiene 10 gemäß dem Ausführungsbeispiel kann auch die Zahnschiene ohne den Sensor 20, d.h. lediglich der entsprechende Kunststoffkörper 2 aufgefasst werden, wobei die für die Verbindung mit dem Sensor relevanten Teile des Kunststoffkörpers 2 auf den Sensor 20 abgestimmt sind.

Bei dem Sensor 20 gemäß dem Ausführungsbeispiel handelt es sich um einen Thermosensor zur Überwachung der Tragezeit der Zahnschiene 10. Gemäß anderen, nicht in den Figuren dargestellten Ausführungsbeispielen handelt es sich bei dem Sensor um einen Sensor einer anderen Art.

Gestalt und Größe des Sensors 20 gemäß dem Ausführungsbeispiel entspricht üblichen am Markt erhältlichen Thermosensoren. Gemäß anderen, nicht in den Figuren dargestellten Ausführungsbeispielen kann der Sensor anders geformt sein. Typischerweise wird einem flachen Sensor der Vorzug gegeben, weil sich ein flacher Sensor einfacher in einer intraoralen Vorrichtung unterbringen lässt.

Der Kunststoffkörper 2 der Zahnschiene 10 gemäß dem in den Figuren 2 bis 9 dargestellten Ausführungsbeispiel der vorliegenden Erfindung unterscheidet sich von der in Fig. 1 dargestellten konventionellen Zahnschiene dadurch, dass im Backenzahnbereich eine Sensorhalterung 11 vorgesehen ist. Die Sensorhalterung 11 ist in Form einer Vertiefung auf der Seite der Innenoberfläche 3 des Kunststoffkörpers ausgebildet.

Die Vertiefung weist einen Öffnungsabschnitt 13 und einen Aufnahmeabschnitt 14 auf. Der Aufnahmeabschnitt 14 ist ausgebildet, den Sensor 20 vollständig in sich aufzunehmen. Der Öffnungsabschnitt 13 ist jener Abschnitt, der zwischen dem Aufnahmeabschnitt 14 und dem Zahnbogen bzw. dem Zahnfleisch angeordnet ist, wenn die Zahnschiene 10 intraoral getragen wird.

Die Sensorhalterung 11 weist eine Einraststruktur 12 auf, um den Sensor 20 formschlüssig im Aufnahmeabschnitt 14 der Vertiefung halten zu können. In dem in den Figuren dargestellten Ausführungsbeispiel wird die Einraststruktur 12 dadurch bereitgestellt, dass der Öffnungsabschnitt kleiner bemessen ist, als es den Abmessungen des Sensors entsprechen würde. In anderen Worten ist im Bereich des Öffnungsabschnitts eine Wulst 15 vorgesehen, welche in Bezug auf den Aufnahmeabschnitt eine Unterschneidung darstellt. Da sowohl das Material des Kunststoffkörpers 2 als auch der Sensor 20 eine gewisse Flexibilität aufweisen, ist es möglich, den Sensor 20 durch Anwendung einer ausreichend großen Kraft und einer damit einhergehenden Verformung den Sensor 20 in den Aufnahmeabschnitt 14 einzubringen. Wenn die Kraftanwendung beendet wird und sich die Verformung zurückbildet, rastet der Sensor 20 aufgrund der Unterschneidung bzw. Wulst 15 in der Vertiefung ein und wird daher mittels der Sensorhalterung 11 formschlüssig am Kunststoffkörper 2 befestigt.

Die Unterschneidung bzw. Wulst 15 ist in dem in den Figuren 1 bis 9 dargestellten Ausführungsbeispiel entlang des gesamten Umfangs der Öffnung der Vertiefung ausgebildet. In anderen, nicht in den Figuren dargestellten Ausführungsbeispielen ist am Öffnungsumfang der Vertiefung nur abschnittsweise eine Unterscheidung bzw. Wulst vorgesehen.

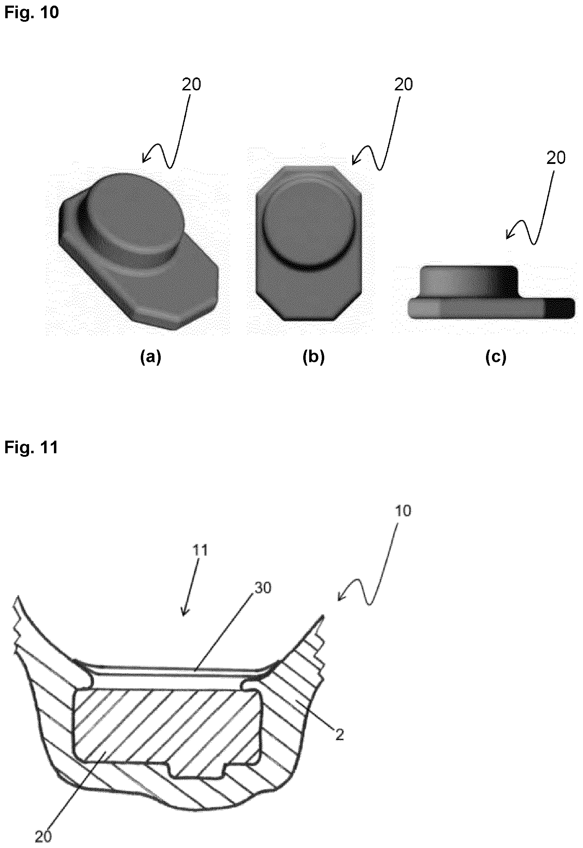

In den Figuren 10 (a) bis 10 (c) ist ein Sensor 20 gemäß einem weiteren Ausführungsbeispiel dargestellt. Er entspricht hinsichtlich der Form etwa dem Sensor 20, der in den Figuren 1 bis 9 dargestellt ist, ist jedoch etwas flacher ausgeführt. Die Sensoren weisen jeweils bevorzugt eine flache Seite auf, welche die dem Mundinneren zugewandte Seite ist.

In Fig. 11 ist ein weiteres Ausführungsbeispiel der vorliegenden Erfindung dargestellt. In diesem Ausführungsbeispiel ist zusätzlich zu dem in den Figuren 2 bis 9 dargestellten Ausführungsbeispiel eine Folie 30 vorgesehen, mit welcher die Vertiefung abgedeckt und vorzugsweise der im Aufnahmeabschnitt 14 befindliche Sensor 20 gegenüber dem Mundinnenraum versiegelt wird. Die Folie ist am Kunststoffkörper 2 befestigt, bevorzugt mit diesem verschweißt.

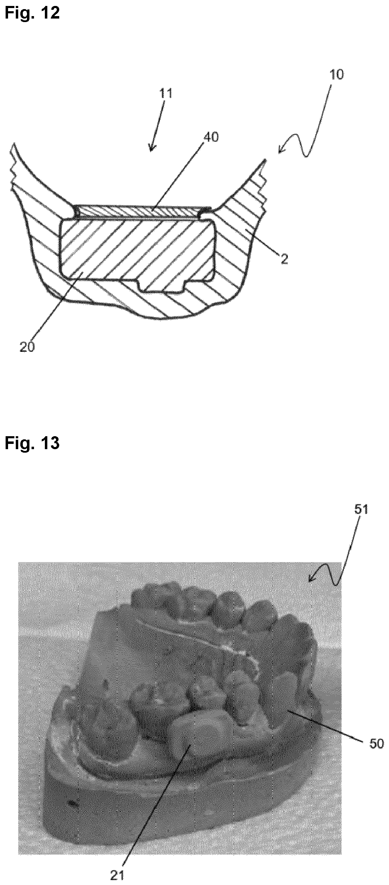

In Fig. 12 ist ein weiteres Ausführungsbeispiel der vorliegenden Erfindung dargestellt. In diesem Ausführungsbeispiel ist zusätzlich zu dem in den Figuren 2 bis 9 dargestellten Ausführungsbeispiel ein Pfropfen 40 vorgesehen, mit welchem die Vertiefung abgedeckt und vorzugsweise der Aufnahmeabschnitt 14 dicht verschlossen und so der im Aufnahmeabschnitt 14 befindliche Sensor 20 gegenüber dem Mundinnenraum versiegelt wird.

Ein Set gemäß einem weiteren Ausführungsbeispiel der vorliegenden Erfindung besteht aus einem Sensor 20 wie für das in den Figuren 2 bis 9 dargestellte Ausführungsbeispiel beschrieben sowie mehreren Zahnschienen 10 (jeweils ohne Sensor) gemäß dem in den Figuren 2 bis 9 dargestellte Ausführungsbeispiel. Bei den Zahnschienen 10 handelt es sich bevorzugt um eine Serie von entsprechend dem das Behandlungsziel und den Behandlungsfortschritt unterschiedlich geformten Zahnschienen für die Aligner-Therapie, die von der Patientin/dem Patienten nacheinander für jeweils einen bestimmten Zeitraum zu tragen sind.

Nachfolgend wird ein Verfahren gemäß einem weiteren Ausführungsbeispiel der vorliegenden Erfindung beschrieben. Mithilfe dieses Verfahrens kann beispielsweise eine Zahnschiene 10 gemäß dem in den Figuren 2 bis 9 dargestellten Ausführungsbeispiel hergestellt werden. Die einzelnen Schritte des Verfahrens sind in einer Reihenfolge beschrieben, in der sie auch zweckmäßigen ausgeführt werden können. Zumindest teilweise kann auch eine andere Abfolge der Schritte sinnvoll sein.

In Schritt A wird ein Sensor 20 bereitgestellt. Bei dem Sensor 20 kann es sich um einen Thermosensor handeln, beispielsweise einen Sensor, der unter dem Handelsnamen "TheraMon" von der Firma MC Technology GmbH in Hargelsberg (Österreich) angeboten wird.

In Schritt B wird ein Abstandshalter entsprechend dem Öffnungsabschnitt 13 der herzustellenden Zahnschiene 10 auf dem Sensor 20 aufgebracht, beispielsweise in Form eines Wachsaufbaus. Der Abstandshalter ist bevorzugt so ausgebildet, dass sich die weiter oben beschriebene Unterschneidung ergibt.

In Schritt C wird ein Abdruck des Sensors mit dem daran aufgebrachten Abstandshalter angefertigt, beispielsweise ein Silicon-Abdruck.

In Schritt D wird anhand des Abdrucks ein Sensormodell 21, welches einstückig mit den Abstandshalter gebildet ist, gefertigt, beispielsweise durch Gießen von Gips.

Alternativ kann ein Sensormodell ohne Abstandshalter angefertigt und auf diesem der Abstandshalter angebracht werden.

Falls bereits ein Sensormodell vorhanden ist, beispielsweise von der Herstellung einer Zahnschiene für eine andere Patientin/einen anderen Patienten, kann auf die Ausführung der vorstehenden Schritte verzichtet und für die nachfolgenden Schritte auf das das bereits vorhandene Sensormodell zurückgegriffen werden.

In Schritt E wird der Abstandshalter des Sensormodells 21 bzw. der am Sensormodell angebrachte Abstandshalter an geeigneter Stelle auf ein Kiefermodell 50 angebracht. Dazu kann beispielsweise Klebstoff dienen. Das Ergebnis des Schritts E, d.h. das Kiefermodell 50 mit dem daran angebrachten Sensormodell 21 einschließlich Abstandshalter, wird nachfolgend als Tiefziehmodell 51 bezeichnet. Ein konkretes Tiefziehmodell ist in Fig. 13 dargestellt.

Im optionalen Schritt F wird eine Platzhalterfolie am Tiefziehmodell 51 tiefgezogen. Als Platzhalterfolie kann beispielsweise eine Polyolefin-Folie, bevorzugt eine Polyethylen-Folie (PE), mehr bevorzugt eine Folie aus Polyethylen mit hoher Dichte (PE-HD) verwendet werden. Ein Beispiel einer geeigneten Platzhalterfolie aus PE-HD ist die von der Firma SCHEU-DENTAL GmbH in Iserlohn (Deutschland) unter dem Handelsnamen "ISOFOLAN" angebotene Folie.

Im Schritt G wird das Material, aus dem der Kunststoffkörper der herzustellenden Zahnschiene 10 bestehen soll und welches nachfolgend als "Schienenmaterial" bezeichnet wird, am Tiefziehmodell 51 mit der darüber befindlichen Platzhalterfolie tiefgezogen.

Als Schienenmaterial kann beispielsweise ein thermoplastischer Polyester, bevorzugt PET-G werden. Ein Beispiel eines geeigneten Schienenmaterials aus PET-G ist das von der Firma SCHEU-DENTAL GmbH in Iserlohn (Deutschland) unter dem Handelsnamen "DURAN" angebotene Material.

Zum Tiefzeihen in den Schritten F und G kann ein in der Zahntechnik übliches Tiefziehgerät verwendet werden. Dabei wird das Tiefziehmodell auf einer Unterlage, beispielsweise einem Granulat, angeordnet. Auf dem Tiefziehmodell wird erweichtes Material (Schienenmaterial und ggf. zuvor eine Platzhalterfolie) angeordnet. Es wird Unterdruck angelegt, sodass das erweichte Material an das Tiefziehmodell gepresst wird. Optional kann das Kiefermodell des anderen Kiefers oder ein anderes geeignetes Werkzeug aufgepresst werden, sodass die Außenoberfläche der Zahnschiene eine zum anderen Zahnbogen passende Form hat.

Im optionalen Schritt H wird der Kunststoffkörper 2 der herzustellenden Zahnschiene grob aus dem Tiefziehprodukt ausgearbeitet, d.h. es werden die überstehenden Teile des Schienenmaterials grob entfernt.

Im Schritt I erfolgt die Trennung des Kunststoffkörpers vom Tiefziehmodell 51 und gegebenenfalls der optionalen Platzhalterfolie. Dabei erweist sich die Verwendung einer Platzhalterfolie als besonders vorteilhaft, weil sie das Sensormodell beim Trennen zurückhält und verhindert, dass das Sensormodell 21 nach dem Trennen in der Sensorhalterung des Kunststoffkörpers verbleibt.

Der Schritt I kann auch vor dem Schritt H erfolgen.

Im Schritt J wird der Kunststoffkörper 2 exakt entsprechend den gewünschten Abmessungen ausgeschnitten.

Der Schritt J kann auch vor dem Schritt I erfolgen.

Im Schritt K wird der Sensor 20 mit dem Kunststoffkörper 2 verbunden, indem der Sensor 20 in die Sensorhalterung 11, die entsprechend den vorstehenden Schritten als Vertiefung ausgebildet ist, eingebracht wird und aufgrund der Unterschneidung bzw. der Wulst 15 einrastet.

Die Erfinderinnen haben erfindungsgemäße Zahnschienen im Selbstversuch getestet. Dabei wurde beispielsweise die Zahnschiene gemäß dem in den Figuren 2 bis 9 dargestellten Ausführungsbeispiel mit bukkaler Anordnung eines Thermosensors im Backenzahnbereich getestet. Diese Zahnschiene wurde mittels des obenstehend beschriebenen Verfahrens hergestellt. Diese Zahnschiene verfügte über keinen Verschluss der als Vertiefung ausgebildeten Sensorhalterung. Die getesteten Zahnschienen wurden jeweils über einen Zeitraum von zwei Wochen täglich 22 Stunden getragen, wobei die Zahnschiene lediglich zum Essen und zur Zahnreinigung aus dem Mund entfernt wurde, nicht jedoch zum Trinken oder bei sportlicher Betätigung. Das Tragen einer Zahnschiene an 22 Stunden pro Tag über zwei Wochen entspricht üblichen Vorgaben im Zuge einer Aligner-Therapie. Im Zuge des Tests traten keine Schleimhautreizungen auf. Ferner konnten im Vergleich zu Zahnschienen ohne Sensor keine zusätzlichen Störgefühle wahrgenommen werden. Ferner löste sich der Sensor während des gesamten Testzeitraums nicht vom Kunststoffkörper. Ferner haben sich im Bereich der Sensorhalterung keine übermäßigen Verunreinigungen angesammelt. Somit ergaben die Tests, dass die erfindungsgemäße Zahnschiene im Hinblick auf die Mundverträglichkeit, den Tragekomfort und die Hygiene gegenüber einer Zahnschiene ohne Sensor keine Nachteile aufweist. Im Unterschied zu einer Zahnschiene ohne Sensor gestattet die erfindungsgemäße Zahnschiene jedoch die Überwachung der Compliance mittels des Sensors.

## Patentansprüche

1. Zahnschiene (10), umfassend einen Kunststoffkörper (2) zum intraoralen Tragen auf zumindest einem Teil der Zähne eines Zahnbogens,
**dadurch gekennzeichnet, dass**
der Kunststoffkörper (2) eine Sensorhalterung (11) aufweist,
wobei die Sensorhalterung (11) ausgebildet ist, einen Sensor (20) reversibel am Kunststoffkörper (2) zu befestigen.

2. Zahnschiene (10) gemäß dem vorstehenden Anspruch,
wobei der Kunststoffkörper (2) zur okklusal bzw. inzisal vollständigen und im Übrigen zumindest teilweisen Aufnahme der Zahnkronen aller Zähne eines Zahnbogens ausgebildet ist,
wobei bevorzugt der Kunststoffkörper (2) zur vollständigen Aufnahme der Zahnkronen aller Zähne eines Zahnbogens ausgebildet ist.

3. Zahnschiene (10) gemäß einem der vorstehenden Ansprüche,
wobei die Zahnschiene (10) eine zur Korrektur von Zahnfehlstellungen ausgebildete Zahnschiene (10), bevorzugt eine Aligner-Schiene (10) ist.

4. Zahnschiene (10) gemäß einem der Ansprüche 1 bis 2,
wobei die Zahnschiene (10) eine zur Aufhellung von Zähnen oder zur Behandlung von Bruxismus oder zur Behandlung schlafbezogener Atemfehlfunktionen, insbesondere zur Behandlung des Schnarchens und/oder der Schlafapnoe, ausgebildete Zahnschiene (10) oder eine Retentionsschiene (10) ist.

5. Zahnschiene (10) gemäß einem der vorstehenden Ansprüche,
wobei die Sensorhalterung (11) eine Vertiefung im Kunststoffkörper (2) mit einer auf einer Oberfläche des Kunststoffkörpers (2) angeordneten Öffnung umfasst, insbesondere in Form einer derartigen Vertiefung ausgebildet ist,
wobei die Vertiefung zur zumindest teilweisen Aufnahme des Sensors (20) ausgebildet ist,
wobei bevorzugt die Vertiefung zur vollständigen Aufnahme des Sensors (20) ausgebildet ist,
wobei mehr bevorzugt die Öffnung der Vertiefung in einem Abschnitt der Oberfläche des Kunststoffkörpers (2) angeordnet ist, die dem Zahnbogen bzw. dem Zahnfleisch zugewandt ist, wenn die Zahnschiene (10) intraoral getragen wird,
wobei noch mehr bevorzugt die Öffnung der Vertiefung in einem Abschnitt der Oberfläche des Kunststoffkörpers (2) angeordnet ist, der sich in einem Backenzahnbereich befindet, wenn die Zahnschiene (10) intraoral getragen wird, wobei am meisten bevorzugt die Öffnung der Vertiefung in einem Abschnitt der Oberfläche des Kunststoffkörpers (2) angeordnet ist, der sich in einem bukkalen Backenzahnbereich befindet, wenn die Zahnschiene (10) intraoral getragen wird.

6. Zahnschiene (10) gemäß einem der vorstehenden Ansprüche,
wobei die Sensorhalterung (11) zur formschlüssigen Verbindung mit dem Sensor (20) ausgebildet ist,
wobei bevorzugt die Sensorhalterung (11) eine Einraststruktur (12) für den Sensor umfasst.

7. Zahnschiene (10) gemäß einem der vorstehenden Ansprüche,
wobei die Vertiefung einen der Form und der Größe des Sensors (20) entsprechenden Aufnahmeabschnitt (14) zur vollständigen Aufnahme des Sensors aufweist,
wobei die Vertiefung ferner einen Öffnungsabschnitt (13) aufweist, der zwischen dem Aufnahmeabschnitt (14) und dem Zahnbogen bzw. dem Zahnfleisch angeordnet ist, wenn die Zahnschiene (10) intraoral getragen wird,
wobei bevorzugt der Öffnungsabschnitt (13) so ausgebildet ist, dass der im Aufnahmeabschnitt (14) angeordnete Sensor (20) den Öffnungsabschnitt (13) ohne Verformung des Sensors (20) und/oder des Kunststoffkörpers (2) nicht passieren kann,
wobei mehr bevorzugt im Bereich des Öffnungsabschnitts (13) eine Einraststruktur (12) für den Sensor ausgebildet ist, insbesondere in Form einer Einraststruktur (12), die bestimmt ist, den Rand eines im Aufnahmeabschnitt (14) angeordneten Sensors (20) dicht einzuschließen,
wobei am meisten bevorzugt die Einraststruktur (12) als Wulst (15) ausgebildet ist, durch die ein im Aufnahmebereich (14) befindlicher Sensor 20 in der Vertiefung einrastet und so formschlüssig am Kunststoffkörper 2 befestigt wird, wobei die Wulst (15) insbesondere entlang des gesamten Umfangs des Öffnungsbereichs (13) verläuft.

8. Zahnschiene (10) gemäß einem der vorstehenden Ansprüche,
wobei der Kunststoffkörper (2) und/oder der Sensor (20) durch Einwirkung einer Kraft zumindest abschnittsweise so elastisch verformbar ist, dass der Sensor (20) zumindest teilweise in die Vertiefung eingeführt werden kann und nach Beendigung der Krafteinwirkung eine formschlüssige Verbindung zwischen dem Sensor (20) und dem Kunststoffkörper (2) besteht.

9. Zahnschiene (10) gemäß einem der vorstehenden Ansprüche,
wobei der Kunststoffkörper (2) thermoplastischen Kunststoff umfasst, bevorzugt ausgewählt aus der aus thermoplastischem Polyester, Polyolefinen, thermoplastischen Elastomermaterialien und thermoplastischen Siliconmaterialien bestehenden Gruppe, mehr bevorzugt einen thermoplastischen Polyester, noch mehr bevorzugt Polyethylenterephthalat-Glycol Copolyester, am meisten bevorzugt Polyethylenterephthalat-Glycol Copolyester (PET-G) ohne Hilfs- und Füllstoffe umfasst, und/oder
wobei der Kunststoffkörper (2) Kunststoff mit einem Zug-Elastizitätsmodul nach ISO 527 von 1000 MPa oder weniger, bevorzugt 600 MPa oder weniger, mehr bevorzugt 500 MPa oder weniger umfasst.

10. Zahnschiene (10) gemäß einem der vorstehenden Ansprüche,
wobei die Zahnschiene (10) ferner umfasst:
einen Verschluss (30, 40) zum Verschließen des in der Sensorhalterung angeordneten Sensors, bevorzugt zum Versiegeln des Aufnahmeabschnitts (14) der als Vertiefung ausgebildeten Sensorhalterung (11) gegenüber dem Mund, wenn die Zahnschiene (10) intraoral getragen wird,
wobei mehr bevorzugt der Verschluss als Pfropfen (40) oder als Folie (30) ausgebildet ist,
wobei noch mehr bevorzugt der Verschluss als Folie (30) ausgebildet ist, die im Bereich der Sensorhalterung (11) einrastet, bevorzugt zwischen einem Bereich des Kunststoffkörpers (2) und dem Sensor (20) eingeklemmt ist, und/oder die mit dem Kunststoffkörper (2) verschweißbar oder verschweißt ist.

11. Zahnschiene (10) gemäß einem der vorstehenden Ansprüche,
wobei die Sensorhalterung (11) in einem Abschnitt des Kunststoffkörpers (2) angeordnet ist, der sich in einem Backenzahnbereich befindet, wenn die Zahnschiene (10) intraoral getragen wird, und/oder
wobei die Sensorhalterung (11) so angeordnet ist, dass der Sensor (20) bukkal angeordnet ist, wenn die Zahnschiene (10) intraoral getragen wird.

12. Zahnschiene (10) gemäß einem der vorstehenden Ansprüche,
weiter umfassend den Sensor (20), welcher mittels der Sensorhalterung (11) am Kunststoffkörper (2) reversibel befestigt ist.

13. Set umfassend eine Zahnschiene (10) oder mehrere Zahnschienen (10) gemäß einem der Ansprüche 1 bis 11 und einen Sensor (20).

14. Verfahren zur Herstellung einer Zahnschiene (10), umfassend die Schritte:
- Bereitstellen eines Kiefermodells (50),
- Bereitstellen eines Sensormodells (21),
- Verbinden des Sensormodells (21) mit dem Kiefermodell (50),
- Bereitstellen eines thermoplastischen Kunststoffmaterials,
- Tiefziehen des thermoplastischen Kunststoffmaterials auf das Kiefermodell (50) mit dem damit verbundenen Sensormodell (21).

15. Verfahren gemäß dem vorstehenden Anspruch,
wobei im Schritt des Verbindens des Sensormodells (21) mit dem Kiefermodell (50) das Sensormodell (21) über einen Abstandhalter mit dem Kiefermodell (50) verbunden wird.
